# EUROPEAN PATENT APPLICATION

(11) **EP 0 728 480 A1**
(43) Date of publication of application: **28.08.1996**
(21) Application number: 96300439.5
(22) Date of filing: 23.01.1996
(51) Int. Cl.: A61K 31/445

(54) **Use of ifenprodil for treatment of elevated intraocular pressure**

(30) Priority: 24.02.1995 JP 36661/95
(71) Applicant: ROHTO PHARMACEUTICAL CO., LTD., Ikuno-ku Osaka-shi Osaka-fu (JP)
(72) Inventor: Miyazaki, Hirohisa, Ikuno-ku, Osaka-shi, Osaka-fu (JP); Fujinaga, Kumiko, Appex Haitsu Yuni 505, Higashiosaka-shi, Osaka-fu (JP); Tanaka, Hitoshi, Kitakatsuragi-gun, Nara-ken (JP)
(74) Representative: Stuart, Ian Alexander

(57) **Abstract**

Use in the manufacture of a composition for reducing elevated intraocular pressure of the compound of the formula (1): 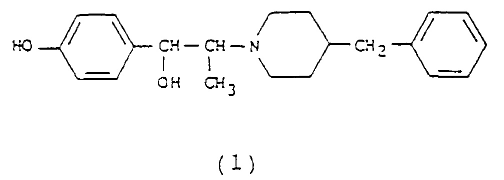
or a pharmaceutically acceptable salt thereof. A pharmaceutical formulation containing the compound of the formula (1) (or salt) as an essential component is also provided, particularly as eye drops.

## Description

The present invention relates to an agent which is useful for the treatment of ocular hypertension (elevated intraocular pressure), and glaucoma. More specifically, this invention relates to a pharmaceutical composition for reducing elevated intraocular pressure which comprises as an essential component 2-(4-benzylpiperidino)-1-(4-hydroxyphenyl)-1-propanol (ifenprodil) of the following formula (1) or any pharmaceutically acceptable salt thereof.

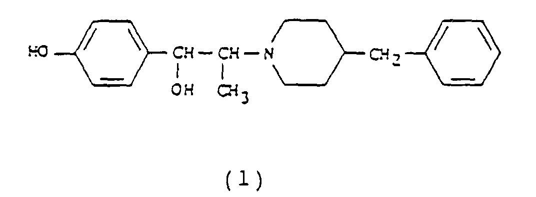

The present invention also relates to a method of reducing elevated intraocular pressure, which comprises administering the compound (I) to patients suffering from abnormally elevated intraocular pressure.

Glaucoma is a disease in which the intraocular pressure (IOP) is persistently or recurrently elevated above the normal range of pressure, which gives organic damage to ocular structures and further impairment of visual function which leads to, for instance, reduction of visual field. "Ocular hypertension" herein used is the term coined to denote an elevated intraocular pressure above the normal level, which is not accompanied by any functional impairment of vision, but, after a long period of time, may develop glaucoma. The medical treatment of ocular hypertension or glaucoma is directed to the reduction of the elevated intraocular pressure down to the normal IOP that induces no functional impairment, and also directed to the maintenance of the normal IOP.

In the present specification, an agent which is useful for reducing elevated intraocular pressure which is found in the ocular hypertension and glaucoma may sometimes be referred to as "antiglaucoma agent" for simplicity.

Conventional antiglaucoma agents known to be effective in the treatment of ocular hypertension or glaucoma are: carbonic anhydrase inhibitors (oral administration), hyperosmotic agents (injection), pilocarpine (eye drops), epinephrine and its prodrug dipivefrine (eye drops), isopropylunoprostone (eye drops). Recently, β-blockers (eye drops) are being extensively used for this purpose. However, all these agents have many disadvantages. Carbonic anhydrase inhibitors, for example, are known to have side effects such as gastrointestinal disturbances and general malaise. Hyperosmotic agents are used mainly in the treatment of acute attacks due to postoperative sudden rise in IOP and are not appropriate for the long-term therapy for glaucoma or ocular hypertension.

Pilocarpine eye drops are known to have several side effects such as feeling of obfuscation due to miosis-induced arctation of the visual field and accommodation disorders due to contraction of the ciliary muscle. Epinephrine and dipivefrine eye drops induce rebound congestion due to vasoconstriction, ophthalmalgia and tachycardia. Isopropylunoprostone eye drops have undesirable side-effects, such as conjunctiva afflux and eye irritation. The eye drops containing β-blockers as an active ingredient have been reported to cause systemic side effects such as headache and depression through their CNS (central nervous system) action, asthmatic symptoms by acting on the respiratory system and bradycardia and hypotension through its cardiovascular action (Iyaku Journal (Medicine and Drug Journal) 28: 705, 1992). When applied topically, β-blockers produce a feeling of dryness due to reduced lacrimal fluid and irritation at the time of instillation. These β-blockers are disadvantageous in that they are contraindicated for patients with bradycardia or bronchial asthma because the above side effects are particularly augmented in these patients (American Journal of Ophthalmology 102: 606, 1986). As described above, none of these antiglaucoma agents now in widespread use are satisfactory.

It is one objective of this invention to provide a novel antiglaucoma agent for treating ocular hypertension or glaucoma, which agent possesses an excellent ocular hypotensive effect (i.e., intraocular pressure-reducing effect) and no significant side effects, in particular, a minimal eye irritating effect.

As a result of extensive study seeking for excellent pharmaceutical agents for treating ocular hypertension, the present inventors have found that the compound (1) known as a therapeutic agent for improving cerebral blood flow and metabolism has an excellent ocular hypotensive effect, and minimal eye irritating effect, and therefore, the compound (1) is useful for the treatment of ocular hypertension or glaucoma.

The pharmacological properties of the compound (1) suggest that the compound (1) has no side effects such as miosis and accommodation disorders found in pilocarpine eye drops or rebound congestion due to vasoconstriction found in epinephrine and dipivefrine eye drops, and conjunctiva afflux and eye irritation found in isopropylunoprostone eye drops. The compound (1) has no side effects such as bradycardia and is free of side-effect on respiratory system, which are found in β-blocker used as eye drops.

As stated above, the compound (1) is a highly useful agent for the treatment of ocular hypertension or glaucoma without having significant side effects possessed by conventional agents.

Compound (1) can be synthesized by referring, for example, to the published literatures listed under "4821. Ifenprodil" on page 777 in the 11th Edition of Merck Index. A commercially available compound (1) can also be used (SIGMA CHEMICAL COMPANY).

Examples of pharmaceutically acceptable salts of the compound (1), which can be the antiglaucoma agent of the present invention, are those formed with inorganic and organic acids, such as hydrochloride, sulfate, nitrate, phosphate, hydrobromate, tartrate, acetate, citrate, fumarate, maleate and oxalate.

The antiglaucoma agent of the present invention can be formulated in a pharmaceutical composition having an appropriate unit dosage form by mixing the compound of the formula (1) or a pharmaceutically acceptable salt thereof with conventional vehicles for pharmaceutical use. The unit dosage form can be any one of conventional dosage forms. Examples of the dosage forms for topical administration may be eye drops and eye ointments, and those for systemic administration may be tablets, granules and injections. It is particularly desirous to use the antiglaucoma agent of the present invention in the form of eye drops.

When used as eye drops, it is desirable to prepare the formulation which contains the compound (1) at a concentration ranging preferably from 0.005 to 0.5 w/v %, more preferably from 0.05 to 0.5 w/v %. Additives that are usually used in formulating eye drops can be used together with the compound (1). Additives to be used include preservatives such as chlorobutanol, sodium dehydroacetate, benzalkonium chloride, cetylpyridinium chloride, phenethyl alcohol, methyl paraoxybenzoate and benzethonium chloride, buffering agents such as borax, boric acid and potassium dihydrogen phosphate, viscosity-increasing agents such as methylcellulose, hydroxyethylcellulose, carboxymethylcellulose, hydroxypropyl methylcellulose, polyvinyl alcohol, sodium carboxymethylcellulose and chondroitin sulfate, solubilizing agents such as polysorbate 80 and polyoxyethylene hydrogenated castor oil 60, and stabilizers such as disodium edetate and sodium bisulfite. It is desirable that eye drops are isotonic with lacrimal fluid, and, for this reason, isotonicating agents such as sodium chloride, potassium chloride, and glycerin may be added as necessary. The pH of the formulation may be at any point within an ophthalmologically acceptable range and is preferably between pH 5.0 and pH 8.0. For preparing eye ointments, any conventional base such as ophthalmologic white petrolatum, propeto or plastibase may be used. Additives such as liquid paraffin may be used.

The dosage and administration mode of the eye drops of the present invention can vary depending on patients' conditions and age. The usual single dosage, however, is 1 - 2 drops and administered one to four times daily. For ophthalmic ointments, an appropriate amount is placed in the conjunctival sac one to two times daily.

Daily dose of ifenprodil may vary from 0.0025 mg to 4 mg. LD₅₀ values for ifenprodil in male and female mice are 950 mg/kg and 910 mg/kg respectively for oral administration, and those in male and female rats are 552 mg/kg and 538 mg/kg respectively for oral administration.

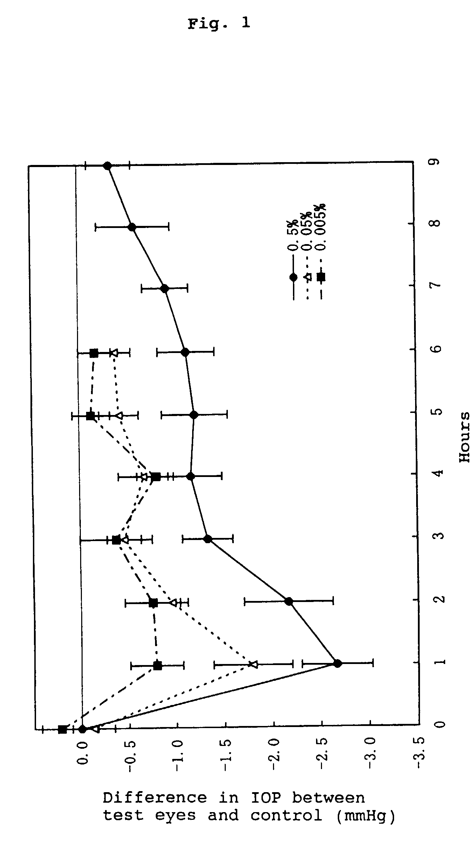

Fig. 1 shows the test results on the major pharmacology (ocular hypotensive effect) of the eye drops of the present invention.

The present invention is explained in more detail below by Examples and Experiments, but this invention is not limited to these Examples and Experiments.

### Example 1

| Eye Drops | |
|---|---|
| Tartrate of Compound (1) | 615 mg |
| (Compound (1) | 500 mg) |
| Glycerin | 2351 mg |
| Benzalkonium chloride | 10 mg |
| Sterile distilled water | appropriate amount |
| Total | 100 ml |

Production: Tartrate of Compound (1) is dissolved in sterile distilled water (80 ml), and glycerin and benzalkonium chloride are added thereto and admixed. Sterile distilled water is added to the mixture to make the total volume 100 ml. pH: 6.5; osmotic pressure-ratio: 1.0.

### Example 2

| Eye Drops | |
|---|---|
| Tartrate of Compound (1) | 62 mg |
| (Compound (1) | 50 mg) |
| Glycerin | 2549 mg |
| Benzalkonium chloride | 10 mg |
| Sterile distilled water | appropriate amount |
| Total | 100 ml |

Production: Eye drops containing the above ingredients are prepared in the similar manner to that described in Example 1. pH: 6.3; osmotic pressure-ratio: 1.0.

### Example 3

| Eye Drops | |
|---|---|
| Tartrate of Compound (1) | 6 mg |
| (Compound (1) | 5 mg) |
| Glycerin | 2568 mg |
| Benzalkonium chloride | 10 mg |
| Sterile distilled water | appropriate amount |
| Total | 100 ml |

Production: Eye drops containing the above ingredients are prepared in the similar manner to that described in Example 1. pH: 6.3; osmotic pressure-ratio: 1.0.

### Example 4

| Eye Drops | |
|---|---|
| Tartrate of Compound (1) | 62 mg |
| (Compound (1) | 50 mg) |
| Boric acid | 1187 mg |
| Borax | 73 mg |
| Sodium Chloride | 283 mg |
| Benzalkonium chloride | 10 mg |
| Sterile distilled water | appropriate amount |
| Total | 100 ml |

Production: Tartrate of Compound (1), boric acid, and borax are dissolved in sterile distilled water (80 ml), and then sodium chloride and benzalkonium chloride are added thereto and admixed. Sterile distilled water is added to the mixture to make the total volume 100 ml. pH: 7.0; osmotic pressure-ratio: 1.0

### Example 5

| Eye Drops | |
|---|---|
| Tartrate of Compound (1) | 62 mg |
| (Compound (1) | 50 mg) |
| Potassium dihydrogen phosphate | 360 mg |
| Disodium hydrogen phosphate | 571 mg |
| Sodium Chloride | 403 mg |
| Benzethonium chloride | 10 mg |
| Sterile distilled water | appropriate amount |
| Total | 100 ml |

Production: Tartrate of Compound (1), potassium dihydrogen phosphate, disodium hydrogen phosphate are dissolved in sterile distilled water (80 ml), and then sodium chloride and benzethonium chloride are added thereto and admixed. Sterile distilled water is added to the mixture to make the total volume 100 ml. pH: 7.0; osmotic pressure-ratio: 1.0

### Example 6

| Eye Drops | |
|---|---|
| Tartrate of Compound (1) | 62 mg |
| (Compound (1) | 50 mg) |
| Liquid paraffin | appropriate amount |
| Total | 100 g |

Production: Eye drops containing the above ingredients are prepared sterilely in the following manner. Tartrate of Compound (1) is ground in a mortar, small amount of liquid paraffin is then added thereto and admixed thoroughly. The mixture is transferred to a dry container, the mortar is washed with small amount of liquid paraffin and remainder of liquid paraffin is then added to the container to make the total weight 100 g. The resultant mixture is stirred to obtain a homogeneous mixture.

### Example 7

| Eye Ointment | |
|---|---|
| Tartrate of Compound (1) | 123 mg |
| (Compound (1) | 100 mg) |
| Liquid paraffin | 5 g |
| Ophthalmologic white petrolatum | appropriate amount |
| Total | 100 g |

Production: Eye ointment containing the above ingredients is prepared sterilely in the following manner. Tartrate of Compound (1) is ground in a mortar, liquid paraffin is then added thereto and admixed thoroughly. The mixture is then admixed with ophthalmologic white petrolatum to obtain an eye ointment.

### Experiment 1: Main pharmacodynamics and pharmacology (ocular hypotensive effect)

The ocular hypotensive effect of the eye drops of this invention was investigated in rabbits. The same experiment was also conducted using a representative β-adrenergic blocking agent, and its ocular hypotensive effect was compared with that of eye drops of this invention.

Methods: According to the formulations described in Examples 1, 2 and 3, three kinds of eye drops respectively containing 0.5, 0.05 and 0.005% of the compound (1) were prepared. As a negative control, eye drops not containing compound (1), which is eye drops described in Reference Example below, were used. The positive control was commercially available eye drops containing timolol maleate (0.5% equivalent of free base) as an active ingredient.

Male Dutch species coloured rabbits (body weight: ca. 2 kg) with normal intraocular pressure were used in the experiment. Each of the test solutions (three kinds of eye drops containing 0.5, 0.05 or 0.005% of ifenprodil) (50 µl) was instilled into one eye of each rabbit, and the control solution free of ifenprodil (50 µl), the eye drops described in Reference Example, into the fellow eye which served as a control. For each solution, six rabbits were used. After anesthesia of the corneal surface by instillating 5 µl of 0.4% oxybuprocaine hydrochloride, the intraocular pressure was measured using an applanation pneumatonograph (Alcon) before and after the instillation of the test solution and control solution. Measurement of intraocular pressure after the instillation was conducted with one-hour intervals until six hours - nine hours post-instillation.

| Reference Example | |
|---|---|
| Glycerin | 2570 mg |
| Benzalkonium chloride | 10 mg |
| 0.1 N Aqueous sodium hydroxide | appropriate amount |
| Sterile distilled water | appropriate amount |
| Total | 100 ml |

Production: Glycerin, benzalkonium chloride and 0.1 N aqueous sodium hydroxide are dissolved in sterile distilled water (90 ml), and then the mixture is added with sterile distilled water to make the total volume 100 ml. pH: 7.0; osmotic pressure-ratio: 1.0

Results: The results of the above test involving the eye drops of the present invention are presented in Fig. 1, wherein difference in IOP between the control and the test eyes are shown. Table 1 compares the test results obtained with the commercial eye drops containing 0.5% timolol maleate as an active ingredient with those obtained with the eye drops of the present invention. The eye drops containing 0.5% of compound (1) produced the maximal IOP reduction one hour after instillation. An average difference of the IOP from the control eye drops was 2.7 mmHg, and this ocular hypotensive effect was retained for nine hours after instillation. The eye drops containing 0.05% of compound (1) produced average 1.8 mmHg IOP reduction one hour after administration. Even eye drops containing 0.005% of compound (1) produced IOP reduction which varies depending on concentration of compound (1).

On the other hand, commercially available timolol maleate eye drops produced average 0.9 mmHg and 0.6 mmHg IOP reduction one and two hours after administration respectively. The eye drops of the present invention having the same concentration of active ingredient (0.5%) produced average 2.7 mmHg and 2.2 mmHg IOP reduction one and two hours after administration respectively. The eye drops of the present invention which contain only 0.05% active ingredient showed more potent IOP reduction than the commercially available eye drops containing timolol maleate.

The above test results shows that the eye drops of the present invention exhibit excellent IOP reduction when compared with eye drops which contain as an essential component typical β-receptor inhibiting agent.

**Table 1.**

| Difference in IOP between the control and test eyes (mmHg) | | | | | |
|---|---|---|---|---|---|
| Drug | Concentration of free base (%) | 1 hr | 2 hr | 4 hr | 6 hr |
| Compound (1) | 0.5 | -2.67 | -2.17 | -1.17 | -1.13 |
| Compound (1) | 0.05 | -1.79 | -0.96 | -0.67 | -0.38 |
| Compound (1) | 0.005 | -0.79 | -0.75 | -0.79 | -0.17 |
| Timolol maleate | 0.5 | -0.88 | -0.58 | -0.25 | -0.13 |

### Experiment 2: Eye irritation test

The eye irritating effect of the eye drops of the present invention was investigated in rabbits.

Methods: According to the descriptions in Experiment 1, various kinds of eye drops containing different concentrations of compound (1) as an active ingredient were prepared, and their eye irritating effects were evaluated by modified Draize test (Gendaino Rinsho (Modern Clinics) 4: 277, 1970).

Male Japanese White rabbits (body weight: ca. 2 kg) were used in the experiment. The eye drops disclosed in Examples 1-3 (100 µl) was instilled into one eye of an animal and commercially available eye drops containing timolol maleate was instilled into the fellow eye eight times with 15 minutes interval. The degree of ocular irritation was assessed by scoring the preocular conditions according to the modified Draize method.

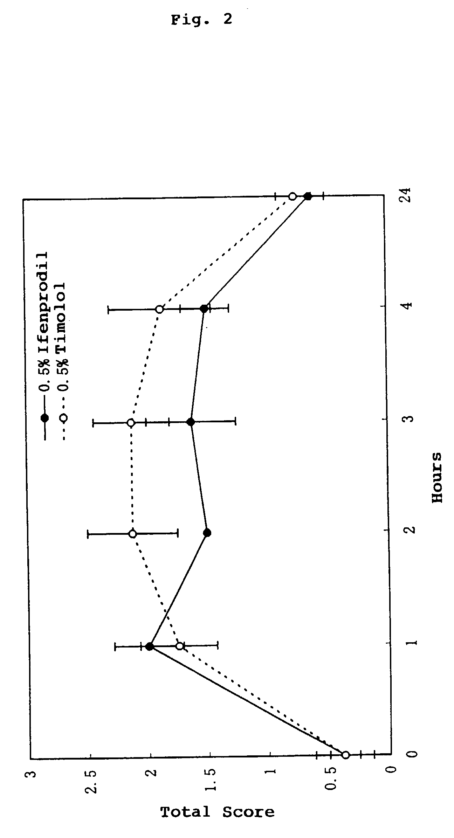

Results: The test results are shown in Fig. 2 with respect to the eye drops containing 0.5% active ingredient. There was observed any significant difference in the scoring between the test eye and the control eye at the time of before administration and 1, 2, 3, 4, and 24 hours after administration. When 0.05% and 0.005% eye drops of the invention were administered, lower scoring was obtained. This shows that the eye drops of the invention give very negligible irritation to eyes.

The pharmaceutical composition of the present invention shows an excellent ocular hypotensive effect and a minimal eye-irritating effect, and these advantages promise that the composition of the present invention is highly useful in the treatment of ocular hypertension and glaucoma.

## Claims

1. Use of the compound of the formula (1): 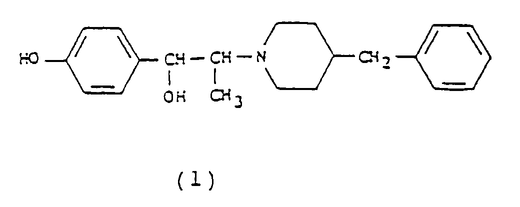 or a pharmaceutically acceptable salt thereof in the manufacture of a pharmaceutical composition for administration to patients suffering from elevated intraocular pressure.

2. The use as claimed in claim 1, wherein the composition is for topical administration.

3. The use as claimed in claim 1 wherein the composition is in the form of eye drops.

4. A pharmaceutical composition for reducing intraocular pressure which comprises as an essential component a compound of the formula (1) as set out in Claim 1 or a pharmaceutically acceptable salt thereof together with a carrier therefor, said composition being adapted for topical application.

5. A pharmaceutical composition according to claim 4 which is in the form of eye drops.

6. A pharmaceutical composition according to claim 5 which contains said compound at a concentration ranging from 0.005 to 0.5 w/v% (or an equivalent amount of a pharmaceutically acceptable salt).

7. A pharmaceutical composition according to claim 5 or 6 containing one or more additives selected from preservatives, buffering agents, viscosity increasing agents, solubilising agents, stabilisers and isotonicating agents.
